# EUROPEAN PATENT APPLICATION

(11) **EP 2 000 528 A1**
(43) Date of publication of application: **10.12.2008**
(21) Application number: 07109499.9
(22) Date of filing: 04.06.2007
(51) Int. Cl.: C12M 1/00

(54) **Shaking apparatus for cell culture incubator or the like**

(71) Applicant: The Automation Partnership (Cambridge) Limited, Royston, Hertfordshire SG8 5WY (GB)
(72) Inventor: Owen, Stephen, Bourn, Hertfordshire CB3 7TZ (GB); Bargh, Adrian Neil, Royston, Hertfordshire SG8 5BW (GB)
(74) Representative: Brunner, Michael John

(57) **Abstract**

An apparatus for shaking a plurality of vessels, for example in an incubator, has at least one rotatable support 10 (101,102) defining a plurality of support locations for vessels 50 at predefined positions 12 around an upright axis and the support or supports 10 are rotatable around the upright axis to move the vessels between a number of positions about the axis for insertion and removal of the vessels onto and from the support(s) respectively. A drive transmission system 38,381 (39,391) connects the support to a drive motor 382 (392), whereby the support can be rotated to a desired position for loading or unloading of a vessel to or from a respective support location 12. A drive mechanism 33, 36 (37). 35 is operable to move each support location eccentrically about an axis independently of the rotation of the support about the upright axis, so that vessels disposed on the support can be shaken. The drive mechanism includes an elongate shaft 33 and the drive transmission system includes a hollow shaft 38 (39) disposed around the elongate shaft of the drive mechanism.

## Description

### BACKGROUND

The invention relates to a system for stirring or shaking vessels which, for example, contain cells which are processed in an incubator of a cell culture system.

Many different types of shaking systems exist. For example, figure-of-eight shakers move in a figure-of-eight pattern and may switch smoothly between clockwise and counterclockwise motion. Hand-motion shakers duplicate the up and down action associated with a handshake. In another type, the entire platform of an orbital shaker moves in a circular orbit. These shakers have many applications in molecular biology including aeration and prevention of "skin" formation. Platforms on reciprocating shakers make back-and-forth "sloshing" motions required for some test procedures. It is known to provide shakers with both an orbital (eccentric) and a reciprocating motion for extra mixing versatility. Platform shakers may provide either a rocking "see-saw" motion or a consistent, three-dimensional rocking motion. Rotating shakers spin samples in test tubes, flasks, or bottles. Wrist-motion shakers mimic the side-to-side action of hand mixing.

For use in incubators, platform shakers are often used, providing one or more trays or shelves on which cell culture vessels can be placed within the incubator, the trays or shelves being capable of being independently shaken. However, such incubators, while they are capable of handling plural cell culture vessels, require manual loading or unloading of the vessels and there is a need for an incubator shaking system which can be loaded robotically.

EP 1626082 A discloses an incubator for example for a cell culture system, the incubator being arranged to handle a plurality of vessels, the incubator including a rotatable support defining a plurality of support locations for vessels at predefined positions around an upright axis and being rotatable around the upright axis to move the vessels between a number of positions about the axis; a drive transmission system for connecting the support to a drive motor, whereby the support can be rotated to a desired position for loading or unloading of a vessel to or from a respective support location; and a drive mechanism operable to move each support location eccentrically about an axis independently of the rotation of the support about the upright axis, whereby vessels disposed on the support can be shaken.

The support locations may be independently eccentrically movable, for example, about individual axes parallel to the upright axis, or alternatively the support is eccentrically movable about the upright axis of the rotatable support whereby the support locations are simultaneously eccentrically movable.

A plurality of independently rotatable supports may be provided, for example disposed one above another, each connectable to the or a respective drive motor. The or each support may be configured to receive vessels in two rings substantially coaxial with the upright axis, one within the other. The rings may be independently rotatable relative to one another.

A robotic handling device can move vessels on to or remove vessels from the or each support as required.

Such an incubator is capable of carrying out the required shaking of multiple culture flasks or vessels as desired and an individual flask may even be omitted from the shaking process by using a robotic handling device to lift the particular flask from its support location during the shaking process.

Shaking devices of this type may find applications beyond incubators.

### SUMMARY OF THE INVENTION

According to the present invention there is provided an apparatus for shaking a plurality of vessels, the apparatus including:
a rotatable support defining a plurality of support locations for vessels at predefined positions around an upright axis and being rotatable around the upright axis to move the vessels between a number of positions about the axis;
a drive transmission system for connecting the support to a drive motor, whereby the support can be rotated to a desired position for loading or unloading of a vessel to or from a respective support location; and
a drive mechanism operable to move each support location eccentrically about an axis independently of the rotation of the support about the upright axis, whereby vessels disposed on the support can be shaken, characterised in that
the drive mechanism includes an elongate shaft and the drive transmission system includes a hollow shaft disposed around the elongate shaft of the drive mechanism.

Additionally, by arranging the elongate shaft to be formed integrally with or carrying a counterbalance, which may extend along most of its length, the possible considerable mass of the oscillating support(s) of the structure, which may be around 150kg in a typical example, can effectively be counterbalanced over the whole height of the apparatus, considerably reducing the transmission of vibration to a surrounding housing and hence adjacent equipment. The mass of the counterbalance can be set at a value sufficient to compensate for both fully unloaded and fully loaded (i.e. all flask location occupied and all flasks filled), as the additional mass of a fully loaded system is, in the same typical example only around 10% of the mass of the supports.

An advantage of such an apparatus is that the elongate shaft may be supported in bearings at both its top and its bottom which provides for a very secure and robust apparatus, necessary particularly when the apparatus has plural supports arranged one above another and the mass of the supports and of filled or partially filled vessels carried by the apparatus may thus be considerable. This enables the use of less massive bearings and results in weight savings and thus reduced material costs.

When, preferably, the apparatus includes plural rotatable supports disposed one above the other, the top and bottom supports are preferably connected for movement around the shaft through eccentric bearings which offset the supports from the axis of the elongate shaft.

The or each support may be configured to receive vessels in two rings substantially coaxial with the elongate shaft, one within the other and the inner and outer rings are preferably independently rotatable relative to one another. The drive transmission system then preferably includes a hollow shaft in driving connection with the inner support ring or rings and a separate, co-axially disposed hollow shaft in driving connection with the outer support ring or rings

Preferably, also, a linkage is provided to prevent the supports precessing around the elongate shaft during its rotation to shake the vessels. This aids in precisely locating the vessels and allows the drive transmission system to be used to its best effect.

### BRIEF DESCRIPTION OF THE FIGURES

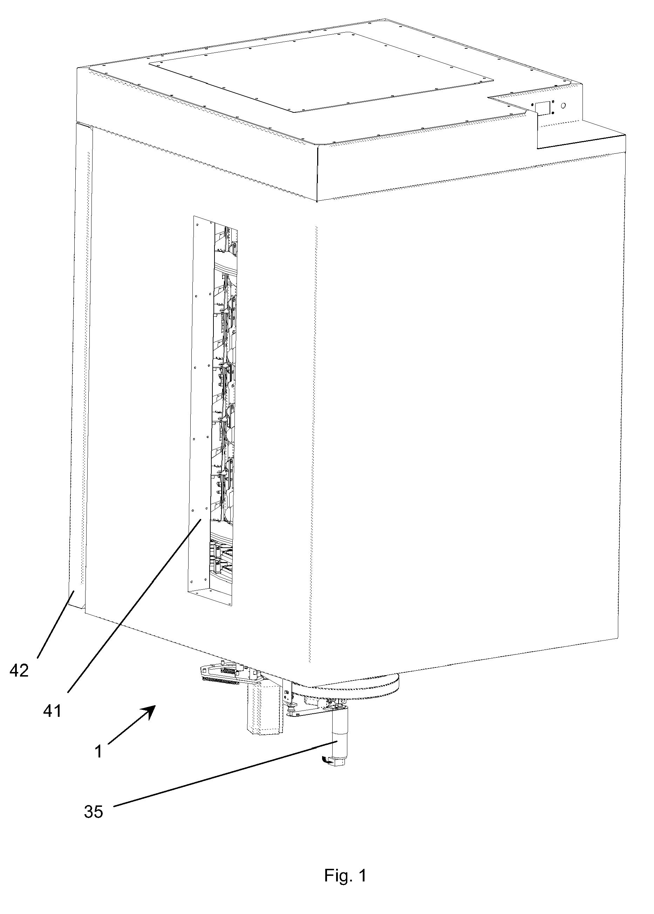

An example of an apparatus according to the present invention will now be described with reference to the accompanying drawings in which:-
Figure 1 is an external isometric view of a shaking apparatus;
Figure 2 is a first section across the apparatus of Figure 1;

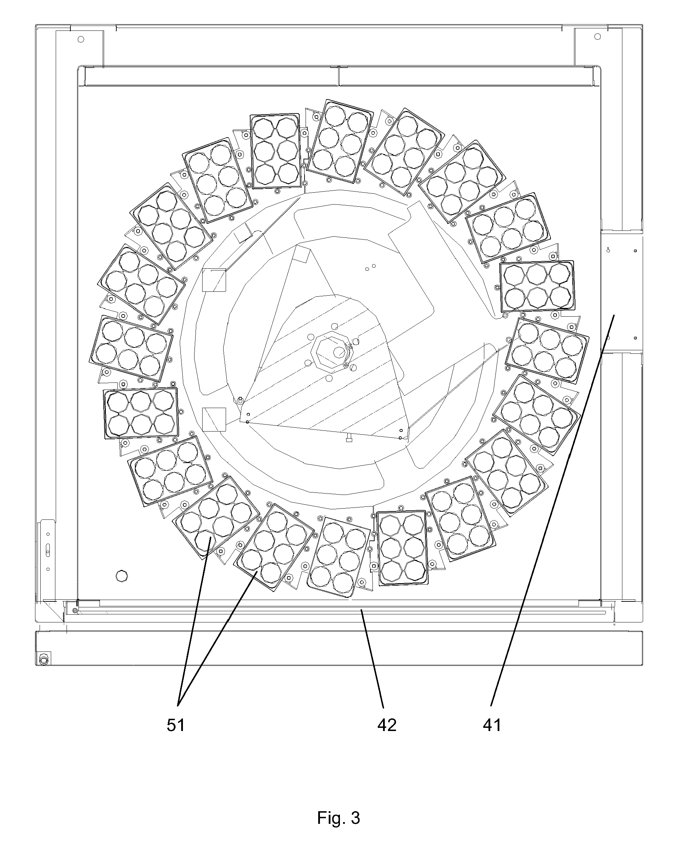

Figure 3 is a second section across the apparatus of Figure 1;
Figure 4 is a third section across the apparatus of Figure 1;

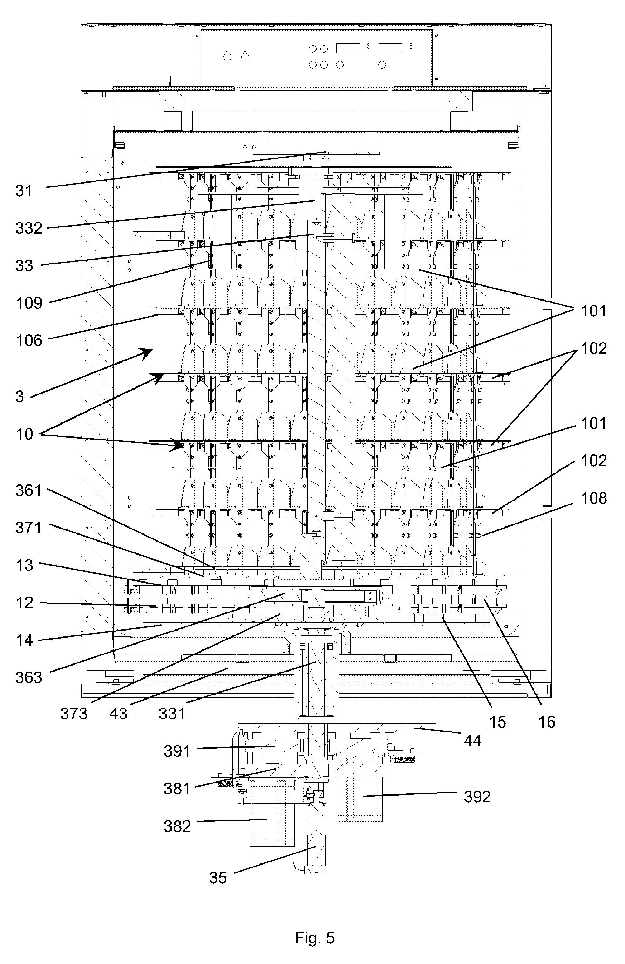

Figure 5 is a vertical section along the centreline of the apparatus of Figure 1;

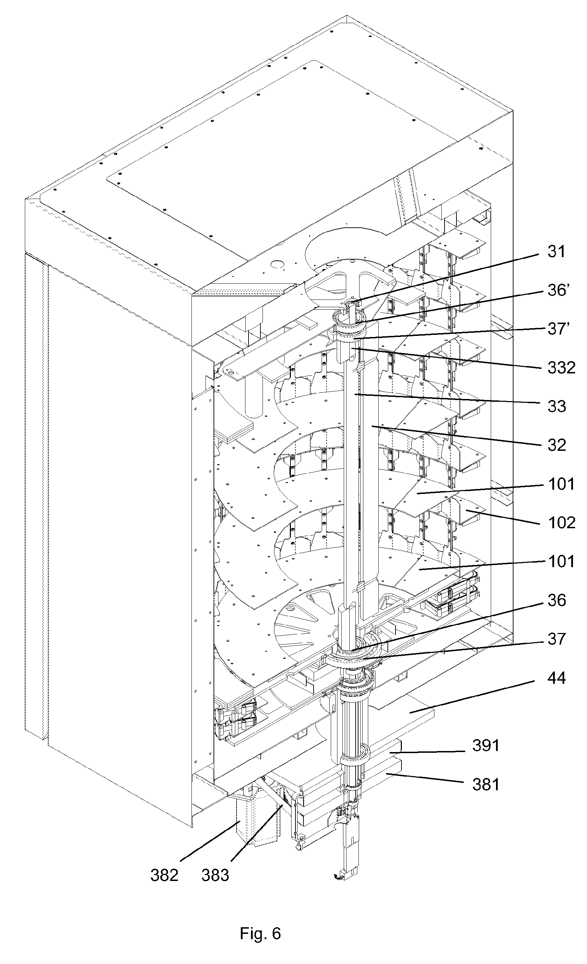

Figure 6 is a partially sectioned isometric view of the apparatus of Figure 1; and

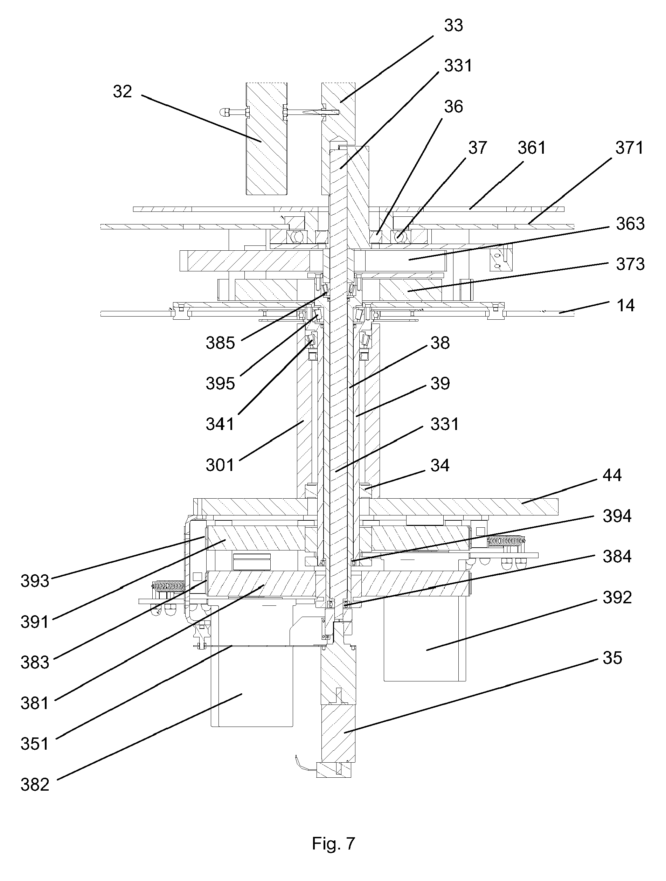

Figure 7 is a vertical section through the lower part of the apparatus of Figure 1, expanded for clarity and with parts of the housing not shown, again for clarity.

### DETAILED DESCRIPTION

The apparatus of this example is described in the context of an incubator 1 which includes a housing 4 in order to enable a controlled environment to be maintained and adjusted. The incubator housing 4 may include a slidable access aperture 41 closable by a door (not shown) through which a robotic arm (not shown) with a gripper unit (also not shown) may be extended to load/unload culture vessels (Erlenmayer flasks) 50 or other vessels such as SBS format microtitre storage plates 51. On the adjacent side, the housing has an operator access door 42. For simplicity further details of the housing of the incubator 1 are not shown in the drawings.

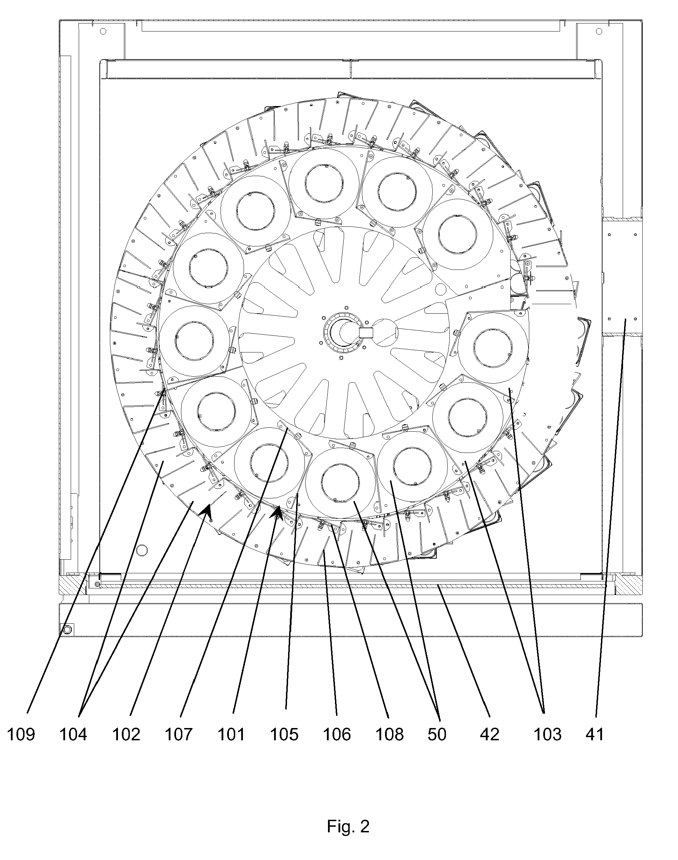

The apparatus 1 includes a carousel 3 providing a first plurality of rotatable supports 10 in the form of pairs of rotatable inner and outer ring-like racks 101, 102 defining, around their common axis, a series of support locations 103,104 for the Erlenmayer flasks 50. Each outer rack 102 is adapted to support flasks of a smaller size (250ml) than the corresponding inner rack 101, which is adapted to support 1 litre E rlenmeyer flasks. The Erlenmeyer flasks 50 are held on the respective racks 101,102 by appropriate spring elements (not shown) which are disposed on the underside of the next rack above the one on which the flask sits and which bear on to the cap of the flask and the flasks are located by respective flask supports 105,107; 109 which, in part, form pockets into which the flask can be positioned. Figure 2 also shows the outer rack 102 and, as the section is taken immediately beneath the next outer ring above, locating brackets 106,108 can be seen which engage with the caps of the flasks on the outer rack in use, the brackets 108 also providing a stop to limit the inward movement of the flasks when they are inserted. Each outer rack 1 02 ha s a section free of spring elements and flask supports in order to allow access therethrough to the respective inner rack 101.

The plural inner racks 101 are fixed for movement together as are the plural outer racks 102. Respective upright flask supports 107,109 extend between respective adjacent racks 101, 102 to support one above another for movement together.

At the top of the incubator housing 4 a bearing 31 is provided which supports the top of an elongate shaft 33 to which is secured, offset, a counterweight 32.

Below the lowest support 10 the incubator includes a floor 43 and below the floor a lower support plate 44 is provided which supports a lower bearing 34 for a lower extension 331 of the elongate shaft 33 within a spacer tube 301. Surrounding the lower extension 331 of the elongate shaft 33 are the first and second tubular index shafts 38,39 which lie between the shaft extension 331 and the bearing 34. The shaft 33,331 is rotated, in use, by an electric motor 35, mounted via a rigid coupling at its lower end, to cause the supports 10 to be shaken in unison by motion in an eccentric movement (around the axis of the shaft 33,331) enabled by the use of a pair of offset bearings 36,37 which are disposed on respective driven plates 361,371 around the upper end of the lower shaft extension 331. The motor is supported on the lower extension 331 of the elongate shaft 33 and a torque arm 351 prevents its rotation when the motor is actuated. The lowermost of the respective support racks 101, 102 are fixed directly to and sit immediately on top of the respective driven plates 361,371 to enable the transfer the eccentric motion of the driven plates to the supports 10 to cause the supports 10 to be shaken when the shaft 33,331 is rotated.

Correspondingly offset bearings 36',37' support the upper most inner and outer support racks 101,102 about an upper shaft extension 332 the shaft 33.

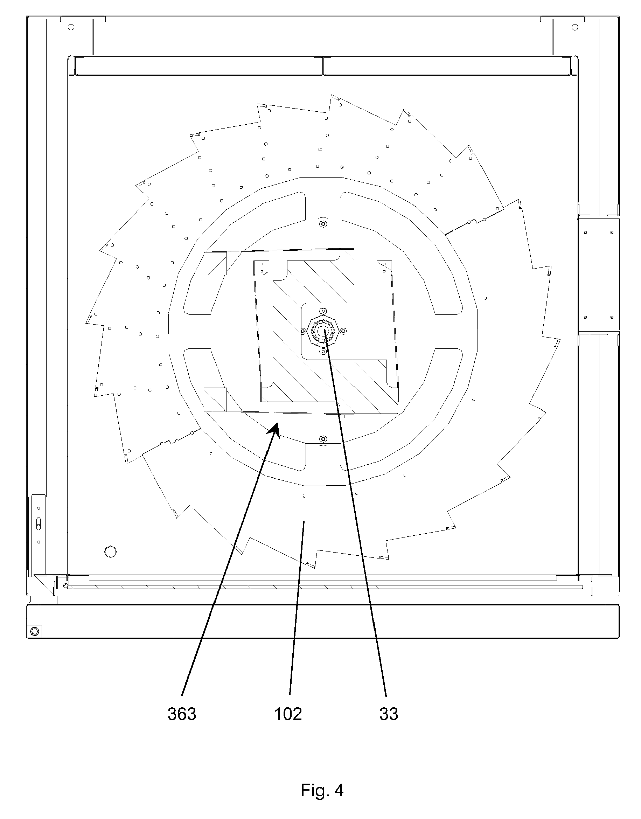

A pair of anti-rotation mechanisms 363,373 are provided to prevent the driven plates 361,371 and hence the supports 10 precessing around the axis of the elongate shaft 33 during shaking. This improves locatability of the individual flasks as the angular position of a flask is then only related to the motor (see below) driving rotation of the support racks as required. The anti-rotation mechanisms 363,373 each comprise a set of leaf springs acting against bearing blocks. Figure 4 shows a cross-section through the anti-rotation mechanism associated with the outer support racks, but as the anti-precession mechanisms form no part of the invention they will not be further described.

Each inner rack 101 is able to rotate within the respective outer rack 102 as the offset bearings 36,37 are aligned with one another so that the axes of offset of the series of inner and outer racks are aligned,

The lower extension 331 of the elongate shaft 33 is surrounded by the first and second tubular index shafts 38,39, as mentioned above, and these are driven through connection to respective drive pulleys 381,391 , and via corresponding toothed drive belts 383,393, by respective stepper motors 382,392 and are indexed to selectively position the inner and outer racks 101,102 as desired. The tubular index shafts are connected to and, on rotation when driven by the motors 382,392, drive the input side of the corresponding inner and outer anti-precession mechanisms 363,373, the output sides of which are respectively connected to the driven plates 361,371, and thus index the respective driven plates and hence the corresponding sets of racks 101,102 of the carousel 3 to position them angularly for loading/unloading of flasks 50. The index shafts 38,39 are supported on respective lower bearings 384,394 and upper bearings 385,395 respectively around the elongate shaft 33 for independent indexing around the axis of rotation of the elongate shaft 33. The bearing 34 also supports the lower ends of the index shafts 38,39 in the lower support plate 44 and a corresponding bearings 341 supports the upper ends of the index shafts 38,39 in the hollow spacer tube 301 within the floor 43 of the incubator housing 4.

Immediately above the floor 43, and surrounding the anti-precession mechanisms 363,373, are disposed a pair of SBS-format plate support rings 12,13 which rotate, when indexed, with the outer support racks 102, but are not shaken. The rings 12,13 are driven from the outer index shaft 39 via a drive plate 14 and two sets of connecting spacer pillars 15,16, between the drive plate 14 and lower support ring 12 and between the lower support ring 12 and the upper support ring 13 respectively. The support rings, 12,13 include spacers 17 to position the SBS-format plates in fixed positions on the support rings 12,13.

It is desirable to determine or fix the position of the support locations when the supports are stationary, before a vessel can be loaded into a support location or removed therefrom by the gripper of the robotic arm. Determining the position may be achieved by means of a suitable sensor, eg. an inductive sensor or a shaft encoder connected to the respective motors 382,392 and the robotic arm may be supplied with data indicative of the position of the support location from the sensor. Alternatively, for example, the supports may be arranged to be brought automatically to a predetermined stop position using feedback from a sensor to a control system operating the respective motor. A further alternative is to provide a biassing system to move the supports to the predetermined position from whatever position they have stopped at.

## Claims

1. An apparatus for shaking a plurality of vessels, the apparatus including:
a rotatable support defining a plurality of support locations for vessels at predefined positions around an upright axis and being rotatable around the upright axis to move the vessels between a number of positions about the axis;
a drive transmission system for connecting the support to a drive motor, whereby the support can be rotated to a desired position for loading or unloading of a vessel to or from a respective support location; and
a drive mechanism operable to move each support location eccentrically about an axis independently of the rotation of the support about the upright axis, whereby vessels disposed on the support can be shaken, **characterised in that**
the drive mechanism includes an elongate shaft and the drive transmission system includes a hollow shaft disposed around the elongate shaft of the drive mechanism.

2. An apparatus according to claim 1, wherein the elongate shaft is integrally formed with or carries a counterbalance.

3. An apparatus according to claim 1 or claim 2, wherein the elongate shaft is supported in bearings at each end.

4. An apparatus according to claim 3, including a plurality of rotatable supports disposed one above the other and wherein the top and bottom supports are connected for movement around the shaft through eccentric bearings.

5. An apparatus according to any of claims 1 to 4, wherein the or each support is configured to receive vessels in two rings substantially coaxial with the elongate shaft, one within the other.

6. An apparatus according to claim 5, wherein the inner and outer rings are independently rotatable relative to one another.

7. An apparatus according to claim 6, wherein the drive transmission system includes a hollow shaft in driving connection with the inner support ring or rings and a separate, co-axially disposed hollow shaft in driving connection with the outer support ring or rings.

8. An apparatus according to any of claims 1 to 7, including a linkage to prevent the supports precessing around the elongate shaft during its rotation to shake the vessels.

9. An incubator including an apparatus according to any of the preceding claims.

10. A cell culture system including an incubator according to claim 8.
